(19) **Europäisches Patentamt / European Patent Office / Office européen des brevets**

(11) **EP 4 067 769 A1**

(12) # EUROPEAN PATENT APPLICATION
published in accordance with Art. 153(4) EPC

(43) Date of publication:
**05.10.2022 Bulletin 2022/40**

(21) Application number: **20894151.8**

(22) Date of filing: **26.11.2020**

(51) International Patent Classification (IPC):
**F24F 11/62** (2018.01)      **F24F 11/64** (2018.01)
**F24F 11/72** (2018.01)      **F24F 11/80** (2018.01)
**F24F 110/10** (2018.01)      **F24F 120/14** (2018.01)
**F24F 120/20** (2018.01)      **F24F 140/00** (2018.01)
**G05B 13/02** (2006.01)      **A61B 5/01** (2006.01)

(52) Cooperative Patent Classification (CPC):
**A61B 5/01; F24F 11/62; F24F 11/63; F24F 11/64;
F24F 11/70; F24F 11/72; F24F 11/80; G05B 13/02;
G06N 20/00;** F24F 2110/10; F24F 2120/14;
F24F 2120/20; F24F 2140/00

(86) International application number:
**PCT/JP2020/044112**

(87) International publication number:
**WO 2021/107053 (03.06.2021 Gazette 2021/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.11.2019   JP 2019213364**

(71) Applicant: **Daikin Industries, Ltd.
Osaka-shi, Osaka 530-8323 (JP)**

(72) Inventor: **NISHIMURA, Tadafumi
Osaka-shi, Osaka 530-8323 (JP)**

(74) Representative: **Global IP Europe
Patentanwaltskanzlei
Pfarrstraße 14
80538 München (DE)**

(54) **MACHINE LEARNING DEVICE AND ENVIRONMENT ADJUSTMENT DEVICE**

(57)      There is provided a machine learning device capable of acquiring a predicted value of a thermal sensation of a subject with a high accuracy. A machine learning device (100) learns a thermal sensation of a subject (20). The machine learning device (100) includes a state variable acquisition unit (101), a control amount acquisition unit (102), and a learning unit (103). The state variable acquisition unit (101) acquires a state variable including a parameter related to biological information of the subject (20). The control amount acquisition unit (102) acquires a control amount including a thermal sensation of the subject (20). The learning unit (103) learns the state variable and the control amount in association with each other.

FIG. 1

EP 4 067 769 A1

**Description**

**TECHNICAL FIELD**

The present disclosure relates to a machine learning device and an environment adjusting apparatus including the same.

**BACKGROUND ART**

PTL 1 (International Publication No. 2007/007632) discloses a configuration that infers the comfort of a subject by performing chaos analysis on time-series data of biological information of the subject and controls an environment adjusting apparatus on the basis of the inferred result.

**SUMMARY OF INVENTION**

< Technical Problem >

There is an issue in that the accuracy of an inferred value of the comfort of a subject is insufficient.

< Solution to Problem >

A machine learning device according to a first aspect learns a thermal sensation of a subject. The machine learning device includes a first acquisition unit, a second acquisition unit, and a learning unit. The first acquisition unit acquires a first variable including a parameter related to biological information of the subject. The second acquisition unit acquires a second variable including a thermal sensation of the subject. The learning unit learns the first variable and the second variable in association with each other.

The machine learning device according to the first aspect can acquire a predicted value of the thermal sensation of the subject with a high accuracy.

A machine learning device according to a second aspect is the machine learning device according to the first aspect, in which the first variable includes at least one of parameters correlated to a brain wave, a skin blood flow rate, a skin temperature, an amount of sweat, and a heartbeat of the subject.

A machine learning device according to a third aspect is the machine learning device according to the first aspect or the second aspect, in which the learning unit performs learning by using, as training data, the first variable and the second variable.

A machine learning device according to a fourth aspect is the machine learning device according to any one of the first to third aspects that further incudes an inference unit. The inference unit infers a predicted value of the thermal sensation of the subject from the first variable on the basis of a learning result of the learning unit.

A machine learning device according to a fifth aspect is the machine learning device according to the fourth aspect that further incudes an updating unit. The updating unit calculates a reward on the basis of the second variable and the predicted value of the thermal sensation of the subject. The learning unit performs learning by using the reward.

A machine learning device according to a sixth aspect is the machine learning device according to the fifth aspect, in which the updating unit calculates a higher reward as a difference between the thermal sensation of the subject included in the second variable and the predicted value of the thermal sensation of the subject becomes smaller.

An environment adjusting apparatus according to a seventh aspect adjusts an environment in a target space. The environment adjusting apparatus includes the machine learning device according to any one of the first to sixth aspects.

An environment adjusting apparatus according to an eighth aspect is the environment adjusting apparatus according to the seventh aspect in which, the second acquisition unit acquires the second variable on the basis of at least one of a value related to the thermal sensation input by the subject and an operation situation of the environment adjusting apparatus.

An environment adjusting apparatus according to a ninth aspect is the environment adjusting apparatus according to the seventh aspect or the eighth aspect that includes an output unit, and a determining unit. The output unit outputs candidates for a third variable for use in adjusting the environment in the target space. The determining unit determines the third variable. The machine learning device is the machine learning device according to any one of the fourth to sixth aspects. The inference unit of the machine learning device infers the predicted value of the thermal sensation of the subject on the basis of the candidates for the third variable output by the output unit. The determining unit determines the third variable such that the predicted value of the thermal sensation of the subject satisfies a predetermined condition.

An environment adjusting apparatus according to a tenth aspect is the environment adjusting apparatus according to the ninth aspect, in which the determining unit determines the third variable such that a difference between a target value of the thermal sensation of the subject and the predicted value of the thermal sensation of the subject inferred by the inference unit decreases.

An environment adjusting apparatus according to an eleventh aspect is the environment adjusting apparatus according to the ninth aspect or the tenth aspect, in which the third variable includes a temperature in the target space.

A machine learning device according to a twelfth aspect learns a control parameter of an environment adjusting apparatus that adjusts an environment in a target space. The machine learning device includes a first acquisition unit, a second acquisition unit, and a learning unit. The first acquisition unit acquires a first variable including a parameter related to biological information of a subject in the target space. The second acquisition unit acquires the control parameter. The learning unit learns the first variable and the control parameter in association with each other.

The machine learning device according to the twelfth aspect can acquire the control parameter of the environment adjusting apparatus suitable for the thermal sensation of the subject.

A machine learning device according to a thirteenth aspect is the machine learning device according to the twelfth aspect that further incudes a third acquisition unit and an updating unit. The third acquisition unit acquires evaluation data for evaluating a control result of the environment adjusting apparatus. The updating unit updates, by using the evaluation data, a learning state of the learning unit. The learning unit performs learning in accordance with an output of the updating unit. The evaluation data includes a thermal sensation of the subject.

A machine learning device according to a fourteenth aspect is the machine learning device according to the thirteenth aspect, in which the updating unit calculates a reward on the basis of the evaluation data. The learning unit performs learning by using the reward.

A machine learning device according to a fifteenth aspect is the machine learning device according to the fourteenth aspect, in which the evaluation data is a difference between a predicted value of the thermal sensation of the subject and a neutral value of a thermal sensation. The updating unit calculates a higher reward as the difference becomes smaller.

A machine learning device according to a sixteenth aspect is the machine learning device according to the thirteenth aspect that further incudes an altering unit. The altering unit outputs a parameter of a discriminant function whose input variable is the first variable and whose output variable is the control parameter. The learning unit alters the parameter of the discriminant function in accordance with an output of the altering unit a plurality of times and outputs, for each discriminant function whose parameter has been altered, the control parameter from the first variable. The updating unit includes an accumulation unit and an assessment unit. The assessment unit outputs an assessment result by using the evaluation data. The accumulation unit accumulates, in accordance with the assessment result, training data based on the first variable and the control parameter output by the learning unit from the first variable. The learning unit performs learning on the basis of the training data accumulated in the accumulation unit.

A machine learning device according to a seventeenth aspect is the machine learning device according to any one of the thirteenth to sixteenth aspects in which, the third acquisition unit acquires the evaluation data on the basis of at least one of a value related to the thermal sensation input by the subject and an operation situation of the environment adjusting apparatus.

A machine learning device according to an eighteenth aspect is the machine learning device according to any one of the twelfth to seventeenth aspects, in which the first variable includes at least one of parameters correlated to a brain wave, a skin blood flow rate, a skin temperature, and an amount of sweat of the subject.

An environment adjusting apparatus according to a nineteenth aspect includes the machine learning device according to any one of the twelfth to eighteenth aspects.

## BRIEF DESCRIPTION OF THE DRAWINGS

Fig. 1 is a block diagram of a machine learning device 100 during learning in accordance with a first embodiment.
Fig. 2 is a block diagram of the machine learning device 100 after learning in accordance with the first embodiment.
Fig. 3 is a block diagram of a machine learning device 100 during learning in accordance with a second embodiment.
Fig. 4 is a block diagram of the machine learning device 100 after learning in accordance with the second embodiment.
Fig. 5 is a block diagram of a machine learning device 200 during learning in accordance with a third embodiment.
Fig. 6 is a block diagram of the machine learning device 200 after learning in accordance with the third embodiment.
Fig. 7 is a block diagram of the machine learning device 200 during learning in accordance with a modification A.
Fig. 8 is a block diagram of the machine learning device 200 after learning in accordance with the modification A.
Fig. 9 is a schematic diagram of a model of a neuron in a neural network.
Fig. 10 is a schematic diagram of a three-layer neural network constituted by a combination of the neurons illustrated in Fig. 9.

Fig. 11 is a diagram for describing a support vector machine, and illustrates a feature space in which pieces of learning data of two classes are linearly separable.

Fig. 12 illustrates a feature space in which pieces of learning data of two classes are linearly inseparable.

Fig. 13 is an example of a decision tree created in accordance with a divide and conquer algorithm.

Fig. 14 illustrates a feature space divided in accordance with the decision tree of Fig. 13.

## DESCRIPTION OF EMBODIMENTS

< First Embodiment >

An environment adjusting apparatus 10 according to a first embodiment will be described with reference to the drawings. The environment adjusting apparatus 10 is an apparatus that adjusts an environment in a target space. In the first embodiment, the environment adjusting apparatus 10 is an air-conditioning control apparatus.

The environment adjusting apparatus 10 predicts a thermal sensation of a subject 20 in the target space by using biological information of the subject 20. On the basis of a predicted value of the thermal sensation of the subject 20, the environment adjusting apparatus 10 grasps the comfort of the subject 20 and implements air-conditioning control for achieving the comfort. The thermal sensation is an index representing the comfort of the subject 20 in the target space. For example, PMV (Predicted Mean Vote) is used as the index of the thermal sensation.

The environment adjusting apparatus 10 includes a machine learning device 100 that learns the thermal sensation of the subject 20 by using a machine learning technique. The machine learning device 100 is constituted by one or a plurality of computers. In the case where the machine learning device 100 is constituted by a plurality of computers, the plurality of computers may be connected to each other via a network.

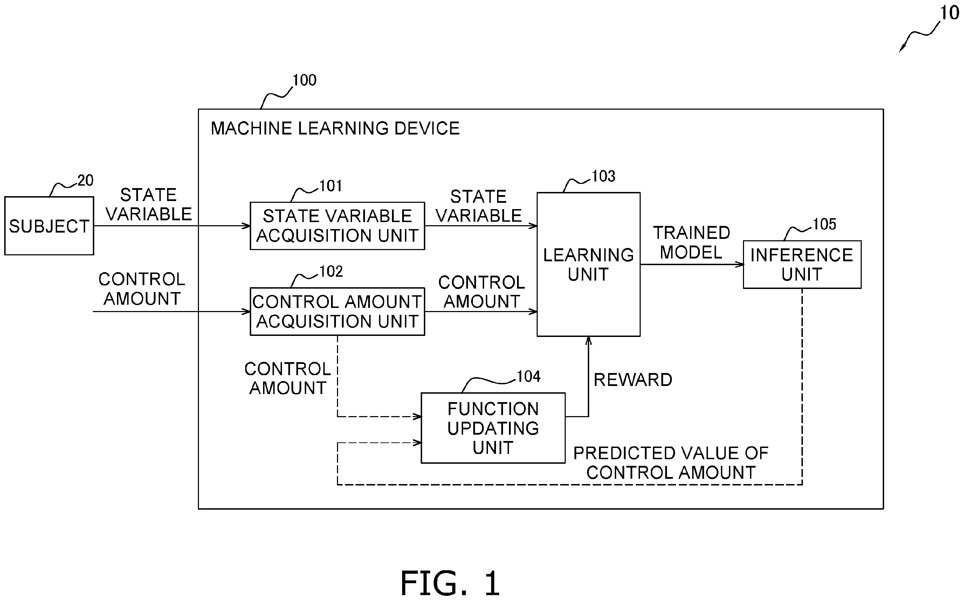

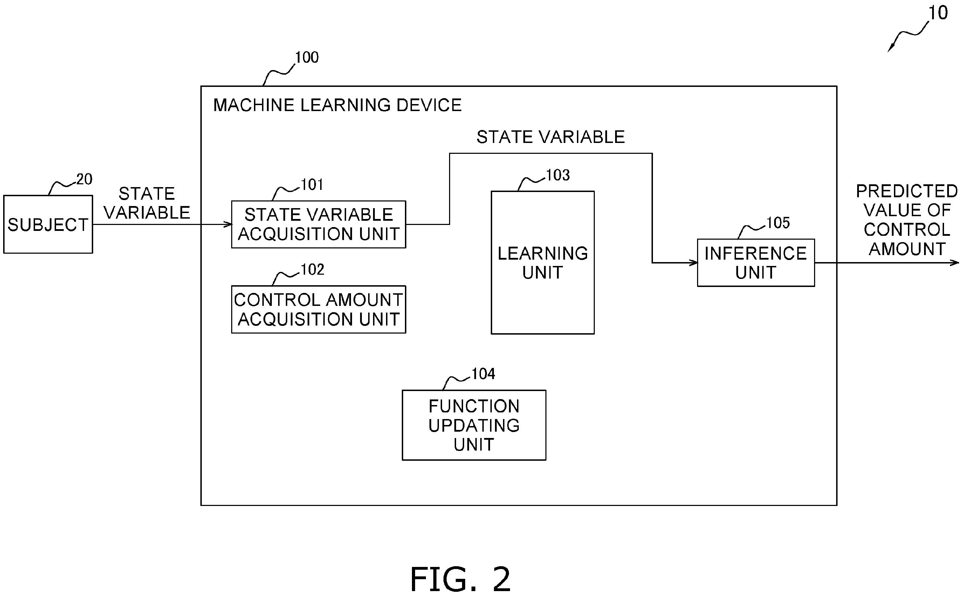

Fig. 1 is a block diagram of the machine learning device 100 during learning in the first embodiment. Fig. 2 is a block diagram of the machine learning device 100 after learning in the first embodiment. The machine learning device 100 mainly includes a state variable acquisition unit 101, a control amount acquisition unit 102, a learning unit 103, a function updating unit 104, and an inference unit 105. The state variable acquisition unit 101 to the inference unit 105 are implemented as a result of a CPU of the machine learning device 100 executing a program stored in a storage device of the machine learning device 100.

The state variable acquisition unit 101 acquires a state variable (first variable) including at least one parameter related to biological information of the subject 20.

The control amount acquisition unit 102 acquires a control amount (second variable) including a thermal sensation of the subject 20.

As illustrated in Fig. 1, the learning unit 103 learns the state variable acquired by the state variable acquisition unit 101 and the control amount acquired by the control amount acquisition unit 102 in association with each other. In the first embodiment, the learning unit 103 performs reinforcement learning in which learning is performed by using a reward. The learning unit 103 outputs a trained model which is a learning result.

The function updating unit 104 calculates the reward on the basis of the control amount acquired by the control amount acquisition unit 102 and a predicted value of the control amount. Specifically, the function updating unit 104 calculates a higher reward as the thermal sensation of the subject 20 included in the control amount is closer to the predicted value of the thermal sensation of the subject 20. That is, the reward calculated by the function updating unit 104 increases as a difference between the actual value of the thermal sensation of the subject 20 and the predicted value of the thermal sensation of the subject 20 decreases.

As illustrated in Fig. 2, the inference unit 105 infers the predicted value of the thermal sensation of the subject 20 from the state variable acquired by the state variable acquisition unit 101, on the basis of the trained model obtained as a result of learning performed by the learning unit 103. The inference unit 105 outputs the predicted value of the thermal sensation of the subject 20. The environment adjusting apparatus 10 performs air-conditioning control on the basis of the predicted value output by the inference unit 105.

The state variable acquired by the state variable acquisition unit 101 includes at least one of parameters correlated to a brain wave, a skin blood flow rate, a skin temperature, an amount of sweat, and a heartbeat of the subject 20. The parameter correlated to a brain wave is at least one of the amplitude of the brain wave, the maximum value of the wave height of the brain wave, and the maximum Lyapunov exponent. The parameter correlated to a skin temperature is at least one of a skin temperature of a specific body portion of the subject 20 and a difference in skin temperature between two specific body portions of the subject 20. The parameter correlated to a heartbeat is, for example, an R-R interval.

The control amount acquisition unit 102 acquires the control amount including the thermal sensation of the subject 20 on the basis of at least one of a value related to the thermal sensation input by the subject 20 and an operation situation of the environment adjusting apparatus 10. The value related to the thermal sensation input by the subject 20 is a thermal sensation based on a subjective vote of the subject 20. For example, the value related to the thermal sensation input by the subject 20 is a thermal sensation input by the subject 20 based on a subjective sensation of the subject 20 and is a thermal sensation calculated from an answer from the subject 20 to a question related to the thermal sensation. The operation situation of the environment adjusting apparatus 10 refers to, for example, a parameter correlated to the brain wave of the subject 20 at the time of the operation of the environment adjusting apparatus 10.

The machine learning device 100 acquires the predicted value of the thermal sensation of the subject 20 by using biological information of the subject 20 which is an objective index. Thus, inclusion of the machine learning device 100 allows the environment adjusting apparatus 10 to acquire the predicted value of the thermal sensation of the subject 20 with a high accuracy. Therefore, the environment adjusting apparatus 10 can implement air-conditioning control for achieving the comfort of the subject 20 on the basis of the predicted value of the thermal sensation of the subject 20.

< Second Embodiment >

An environment adjusting apparatus 10 according to a second embodiment will be described with reference to the drawings. The environment adjusting apparatus 10 according to the first embodiment and the environment adjusting apparatus 10 according to the second embodiment have a common basic configuration. Differences between the first embodiment and the second embodiment will be mainly described below.

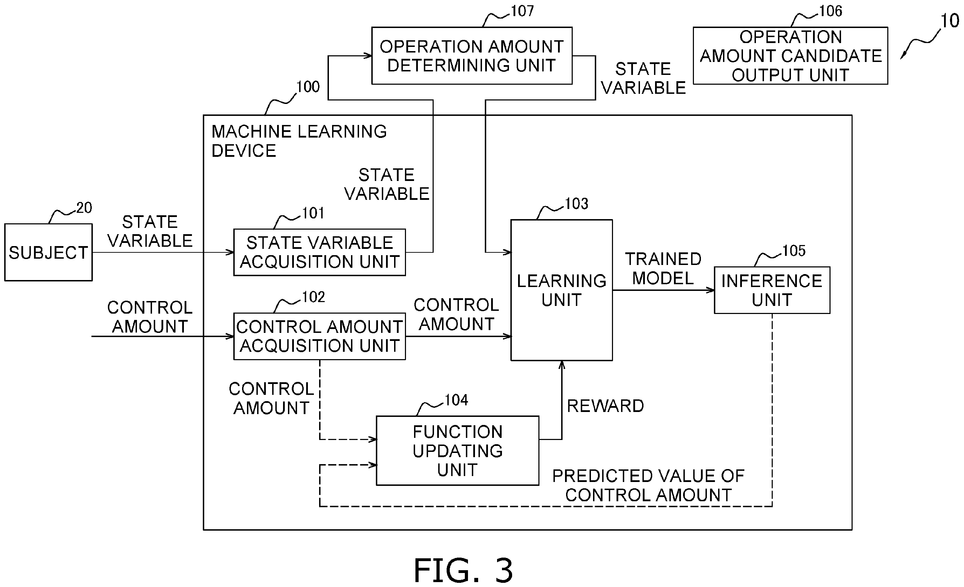

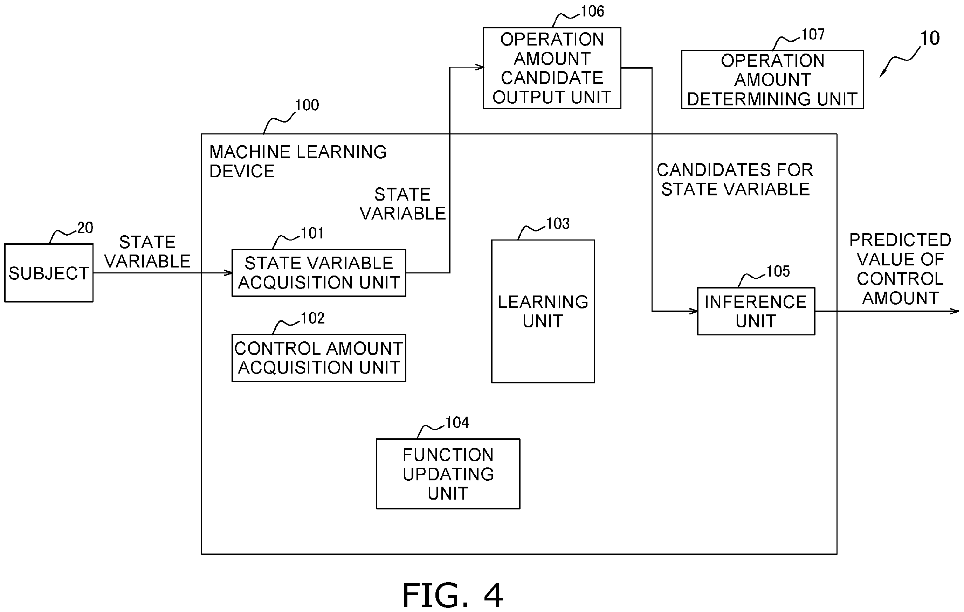

Fig. 3 is a block diagram of a machine learning device 100 during learning in the second embodiment. Fig. 4 is a block diagram of the machine learning device 100 after learning in the second embodiment. The environment adjusting apparatus 10 according to the second embodiment includes the machine learning device 100 according to the first embodiment, an operation amount candidate output unit 106, and an operation amount determining unit 107. The machine learning device 100 includes the state variable acquisition unit 101 to the inference unit 105.

The operation amount candidate output unit 106 outputs candidates for an environmental parameter (third variable) for use in adjusting an environment in a target space. The environmental parameter includes a temperature in the target space. The operation amount candidate output unit 106 outputs candidates for the environmental parameter from a predetermined environmental parameter list, for example. As illustrated in Fig. 4, the inference unit 105 of the machine learning device 100 infers a predicted value of the thermal sensation of the subject 20 on the basis of at least the candidates for the environmental parameter output by the operation amount candidate output unit 106.

The operation amount determining unit 107 determines the environmental parameter such that the predicted value of the thermal sensation of the subject 20 satisfies a predetermined condition. Specifically, the operation amount determining unit 107 determines the environmental parameter such that a difference between a target value of the thermal sensation of the subject 20 and the predicted value inferred by the inference unit 105 decreases. As illustrated in Fig. 3, the learning unit 103 of the machine learning device 100 performs learning by using the environmental parameter determined by the operation amount determining unit 107, and outputs a trained model.

In the second embodiment, from among the candidates for the environmental parameter, the operation amount determining unit 107 can determine the environmental parameter suitable for creating a trained model capable of acquiring the predicted value of the thermal sensation of the subject 20 with a high accuracy. Therefore, the environment adjusting apparatus 10 can acquire the predicted value of the thermal sensation of the subject 20 with a high accuracy and implement air-conditioning control for achieving the comfort of the subject 20 on the basis of the predicted value of the thermal sensation of the subject 20.

< Third Embodiment >

An environment adjusting apparatus 10 according to a third embodiment will be described with reference to the drawings. The environment adjusting apparatus 10 is an apparatus that adjusts an environment in a target space. In the third embodiment, the environment adjusting apparatus 10 is an air-conditioning control apparatus.

The environment adjusting apparatus 10 predicts a thermal sensation of a subject 20 in the target space by using biological information of the subject 20. On the basis of a predicted value of the thermal sensation of the subject 20, the environment adjusting apparatus 10 grasps the comfort of the subject 20 and implements air-conditioning control for achieving the comfort.

The environment adjusting apparatus 10 includes a machine learning device 200 that learns a control parameter of the environment adjusting apparatus 10. The machine learning device 200 is constituted by one or a plurality of computers. In the case where the machine learning device 200 is constituted by a plurality of computers, the plurality of computers may be connected to each other via a network.

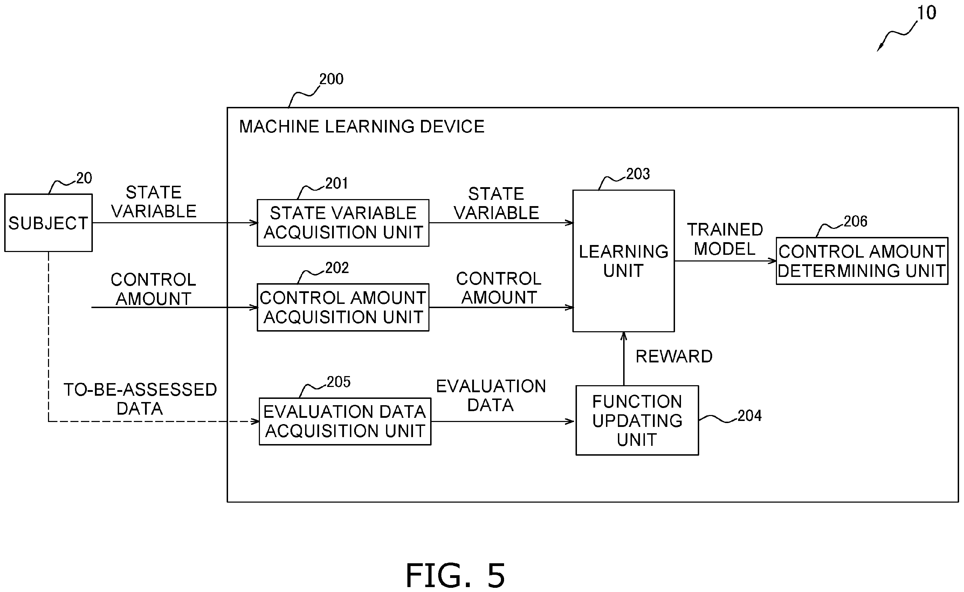

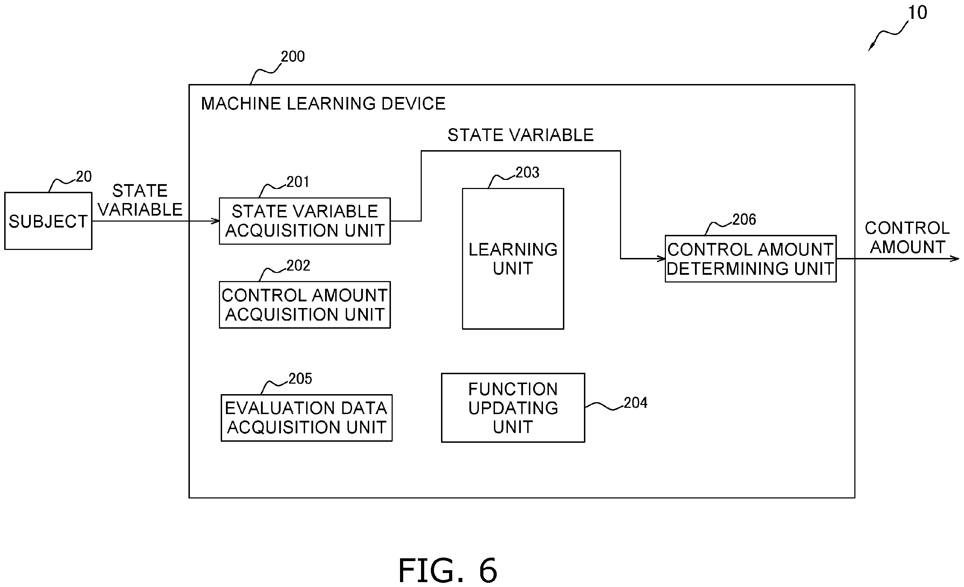

Fig. 5 is a block diagram of the machine learning device 200 during learning in the third embodiment. Fig. 6 is a block diagram of the machine learning device 200 after learning in the third embodiment. The machine learning device 200 mainly includes a state variable acquisition unit 201, a control amount acquisition unit 202, a learning unit 203, a function updating unit 204, an evaluation data acquisition unit 205, and a control amount determining unit 206. The state variable acquisition unit 201 to the control amount determining unit 206 are implemented as a result of a CPU of the machine learning device 200 executing a program stored in a storage device of the machine learning device 200.

The state variable acquisition unit 201 acquires a state variable (first variable) including at least one parameter related to biological information of the subject 20 in the target space.

The control amount acquisition unit 202 acquires, as a control amount, a control parameter of the environment adjusting apparatus 10.

The evaluation data acquisition unit 205 acquires evaluation data for evaluating a control result of the environment adjusting apparatus 10.

The function updating unit 204 updates a learning state of the learning unit 203 by using the evaluation data acquired by the evaluation data acquisition unit 205.

As illustrated in Fig. 5, the learning unit 203 learns the state variable acquired by the state variable acquisition unit 201 and the control parameter acquired by the control amount acquisition unit 202 in association with each other. The learning unit 203 outputs a trained model which is a learning result.

The learning unit 203 performs learning in accordance with an output of the function updating unit 204. In the third embodiment, the learning unit 203 performs reinforcement learning in which learning is performed by using a reward. The function updating unit 204 calculates the reward on the basis of the evaluation data acquired by the evaluation data acquisition unit 205. Specifically, the function updating unit 204 calculates a higher reward as the thermal sensation of the subject 20 is closer to neutral.

As illustrated in Fig. 6, on the basis of the trained model obtained as a result of learning performed by the learning unit 203, the control amount determining unit 206 determines the control parameter of the environment adjusting apparatus 10 from the state variable acquired by the state variable acquisition unit 201. On the basis of the control parameter determined by the control amount determining unit 206, the environment adjusting apparatus 10 performs air-conditioning control.

The evaluation data acquisition unit 205 inputs predetermined to-be-assessed data to a predetermined evaluation function, and acquires an output value of the evaluation function as the evaluation data. That is, the evaluation function receives the to-be-assessed data as an input value from the evaluation data acquisition unit 205, and outputs the evaluation data. The to-be-assessed data is at least one of the value related to the thermal sensation input by the subject 20 and the operation situation of the environment adjusting apparatus 10. The value related to the thermal sensation input by the subject 20 is a thermal sensation based on a subjective vote of the subject 20. For example, the value related to the thermal sensation input by the subject 20 is a thermal sensation input by the subject 20 based on a subjective sensation of the subject 20 and is a thermal sensation calculated from an answer from the subject 20 to a question related to the thermal sensation. The operation situation of the environment adjusting apparatus 10 refers to, for example, a parameter correlated to the brain wave of the subject 20 at the time of the operation of the environment adjusting apparatus 10.

The evaluation data acquired by the evaluation data acquisition unit 205 includes at least the thermal sensation of the subject 20. The evaluation data is, for example, a predicted value of the thermal sensation of the subject 20. The predicted value of the thermal sensation of the subject 20 is acquired from at least one of the value related to the thermal sensation input by the subject 20 and the operation situation of the environment adjusting apparatus 10. The evaluation data may be a difference between the predicted value of the thermal sensation of the subject 20 and a neutral value of a thermal sensation. In this case, the function updating unit 204 calculates a higher reward as the difference, which is the evaluation data acquired by the evaluation data acquisition unit 205, is closer to zero.

The state variable acquired by the state variable acquisition unit 201 includes at least one of parameters correlated to a brain wave, a skin blood flow rate, a skin temperature, and an amount of sweat of the subject 20. The parameter correlated to a brain wave is at least one of the amplitude of the brain wave, the maximum value of the wave height of the brain wave, and the maximum Lyapunov exponent. The parameter correlated to a skin temperature is at least one of a skin temperature of a specific body portion of the subject 20 and a difference in skin temperature between two specific body portions of the subject 20.

The machine learning device 200 acquires the thermal sensation of the subject 20 on the basis of biological information of the subject 20 which is an objective index, and determines the control parameter of the environment adjusting apparatus 10 on the basis of the thermal sensation of the subject 20. Thus, inclusion of the machine learning device 200 allows the environment adjusting apparatus 10 to acquire the control parameter in which the biological information of the subject 20 is directly reflected. Therefore, the environment adjusting apparatus 10 can implement air-conditioning control for achieving the comfort of the subject 20 on the basis of the thermal sensation of the subject 20.

< Modifications >

At least some modifications of the embodiments will be described below.

(1) Modification A

In the third embodiment, the learning unit 203 performs reinforcement learning in which learning is performed by using a reward. However, instead of reinforcement learning, the learning unit 203 may perform supervised learning in which learning is performed on the basis of training data.

An environment adjusting apparatus 10 according to a modification A will be described with reference to the drawings. The environment adjusting apparatus 10 according to the third embodiment and the environment adjusting apparatus 10 according to the modification A have a common basic configuration. Differences between the third embodiment and the modification A will be mainly described below.

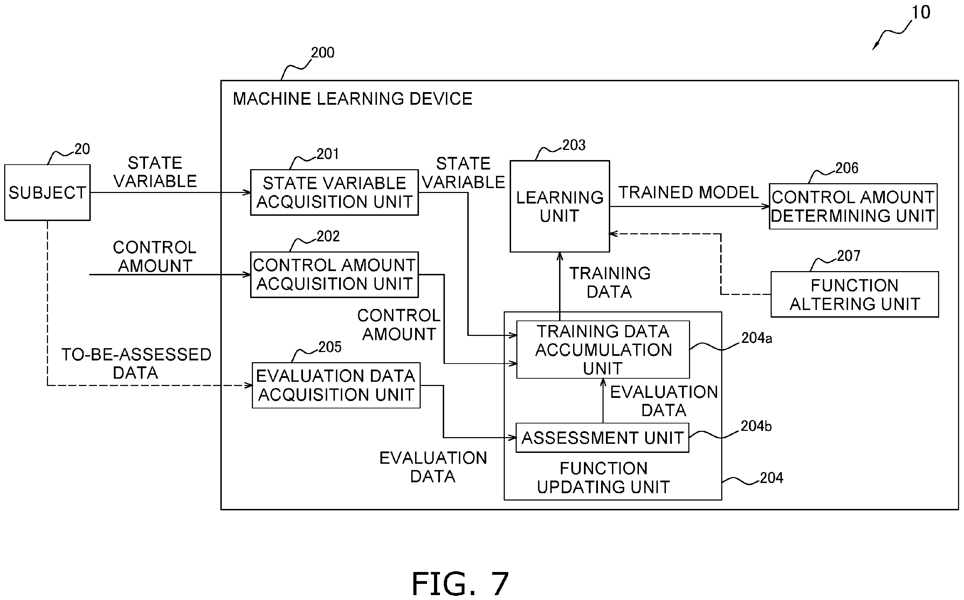

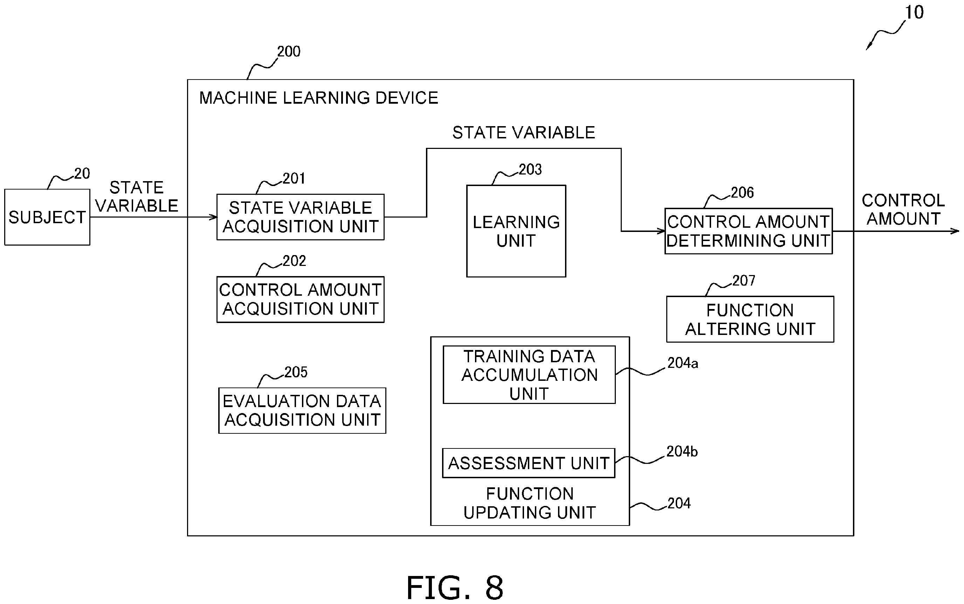

Fig. 7 is a block diagram of a machine learning device 200 during learning in the modification A. Fig. 8 is a block diagram of the machine learning device 200 after learning in the modification A. The machine learning device 200 further includes a function altering unit 207.

The function updating unit 204 includes a training data accumulation unit 204a and an assessment unit 204b. By using the evaluation data acquired by the evaluation data acquisition unit 205, the assessment unit 204b outputs an assessment result of the evaluation data. In accordance with the assessment result obtained by the assessment unit 204b, the training data accumulation unit 204a accumulates training data based on the state variable acquired by the state variable acquisition unit 201 and the control parameter acquired by the control amount acquisition unit 202.

The learning unit 203 slightly alters a parameter of a discriminant function in accordance with the output of the function altering unit 207. The learning unit 203 alters the parameter of the discriminant function a plurality of times and outputs, for each discriminant function whose parameter has been altered, the control parameter from the state variable. The discriminant function refers to a mapping from the state variable included in training data to the control parameter. Specifically, the discriminant function is a function whose input variable is the state variable and whose output variable is the control parameter. The function altering unit 207 outputs the parameter of the discriminant function. If it is determined that the evaluation data obtained as a result of control of the environment adjusting apparatus 10 on the basis of the control parameter output by the learning unit 203 from the state variable is appropriate, the function updating unit 204 accumulates, as training data, the state variable and the control parameter output by the learning unit 203 from the state variable.

The learning unit 203 performs learning on the basis of the training data accumulated in the training data accumulation unit 204a. The purpose of learning performed by the learning unit 203 is to adjust the parameter of the discriminant function by using the training data as learning data so that correct or appropriate evaluation data can be obtained from a new state variable. The learning unit 203 uses, as the learning data, pairs of the state variable acquired in advance by the state variable acquisition unit 201 and the control parameter acquired by the control amount acquisition unit 202. The discriminant function whose parameter is sufficiently adjusted by the learning unit 203 corresponds to the trained model.

The control amount determining unit 206 determines the control parameter from a new state variable on the basis of the trained model obtained as a result of learning performed by the learning unit 203.

As described next, the learning unit 203 performs supervised learning based on online learning or batch learning.

In supervised learning based on online learning, the learning unit 203 generates a trained model in advance by using data (state variable) acquired in a test operation or the like performed before shipment or installation of the environment adjusting apparatus 10. At the time of the start of the initial operation of the environment adjusting apparatus 10, the control amount determining unit 206 determines the control parameter on the basis of the trained model generated in advance by the learning unit 203. The learning unit 203 then updates the trained model by using data (state variable) newly acquired during the operation of the environment adjusting apparatus 10. The control amount determining unit 206 determines the control parameter on the basis of the trained model updated by the learning unit 203. As described above, in the online learning, the trained model is regularly updated, and the control amount determining unit 206 determines the control parameter on the basis of the latest trained mode.

In supervised learning based on batch learning, the learning unit 203 generates a trained model in advance by using data (state variable) acquired in a test operation or the like performed before shipment or installation of the environment adjusting apparatus 10. At the time of the operation of the environment adjusting apparatus 10, the control amount determining unit 206 determines the control parameter on the basis of the trained model generated in advance by the learning unit 203. This trained model is not updated after being generated in advance by the learning unit 203. That is, the control amount determining unit 206 determines the control parameter by using the same trained model.

Note that a server connected to the environment adjusting apparatus 10 via a computer network such as the Internet may generate the trained model, or the trained model may be generated by using a cloud computing service.

(2) Modification B

In the first and second embodiments, the learning unit 103 performs reinforcement learning in which learning is performed by using a reward. However, instead of reinforcement learning, the learning unit 103 may perform supervised learning in which learning is performed on the basis of training data, as described in the modification A. In this case, the learning unit 103 may perform learning by using training data obtained from the state variable acquired by the state variable acquisition unit 101 and the control amount (the thermal sensation of the subject 20) acquired by the control amount acquisition unit 102.

(3) Modification C

In the modifications A and B, in the case where the learning units 103 and 203 perform supervised learning in which training data is used, the learning units 103 and 203 may use part of the training data as learning data to adjust the parameter of the discriminant function and may use the rest of the training data as test data. The test data is data that is not used in learning and is mainly used for evaluation of the performance of the trained model. The use of the test data enables the accuracy of the evaluation data obtained from a new state variable to be predicted in a form of an error probability for the test data. As techniques for splitting pieces of data acquired in advance into learning data and test data, hold-out, cross-validation, leave-one-out (jackknife), bootstrapping, and the like are used.

(4) Modification D

Supervised learning that is a machine learning technique used by the learning units 103 and 203 in the modifications A to C will be described. Supervised learning is a technique for generating an output corresponding to unseen input data by using training data. In supervised learning, learning data and a discriminant function are used. The learning data is a set of pairs of input data and training data corresponding to the input data. The input data is, for example, a feature vector in a feature space. The training data is, for example, parameters regarding discrimination, classification, and evaluation of the input data. The discriminant function represents a mapping from input data to an output corresponding to the input data. Supervised learning is a technique of adjusting a parameter of the discriminant function by using learning data given in advance such that a difference between an output of the discriminant function and training data decreases. Models or algorithms used in supervised learning include a regression analysis, a time-series analysis, a decision tree, a support vector machine, a neural network, ensemble learning, etc.

The regression analysis is, for example, a linear regression analysis, a multiple regression analysis, or a logistic regression analysis. The regression analysis is a technique for applying a model between input data (explanatory variable) and training data (objective variable) by using the least squares method or the like. The dimension of the explanatory variable is 1 in the linear regression analysis and 2 or higher in the multiple regression analysis. In the logistic regression analysis, a logistic function (sigmoid function) is used as the model.

The time-series analysis refers to, for example, an AR model (autoregressive model), an MA model (moving average model), an ARMA model (autoregressive moving average model), an ARIMA model (autoregressive integrated moving average model), an SARIMA model (seasonal autoregressive integrated moving average model), or a VAR model (vector autoregressive model). The AR, MA, ARMA, and VAR models represent a stationary process. The ARIMA and SARIMA models represent a non-stationary process. The AR model is a model in which a value regularly changes as time passes. The MA model is a model in which a fluctuation in a certain period is constant. For example, in the MA model, a value at a certain time point is determined by a moving average before the time point. The ARMA model is a combined model of the AR model and the MA model. The ARIMA model is a model in which the ARMA model is applied to a difference between preceding and following values in consideration of a middle-term or long-term trend (increasing or decreasing trend). The SARIMA model is a model in which the ARIMA model is applied in consideration of a middle-term or long-term seasonal fluctuation. The VAR model is a model in which the AR model is expanded to support multiple variables.

The decision tree is a model for generating complex discrimination boundaries by combining a plurality of discriminators. Details of the decision tree will be described later.

The support vector machine is an algorithm for generating a two-class linear discriminant function. Details of the support vector machine will be described later.

The neural network is obtained by modeling a network that is formed by connecting neurons of the human cranial nervous system by synapses. The neural network means a multilayer perceptron that uses error backpropagation in a narrow sense. The typical neural networks include a convolutional neural network (CNN) and a recurrent neural network (RNN). The CNN is a type of a non-fully-connected (coarsely-connected) forward-propagation neural network. The RNN is a type of the neural network having a directed cycle. The CNN and the RNN are used in audio/image/moving image recognition and natural language processing.

The ensemble learning is a technique for improving the discrimination performance by combining a plurality of models. The technique used in the ensemble learning is, for example, bagging, boosting, or a random forest. Bagging is a technique for training a plurality of models by using bootstrap sampling of learning data and determining evaluation for new input data by a majority vote of the plurality of models. Boosting is a technique for weighting learning data in accordance with a bagging-based learning result, so that incorrectly discriminated learning data is learned in a more concentrated manner than correctly discriminated learning data. The random forest is a technique for generating a decision tree group (random forest) constituted by a plurality of decision trees having a low correlation in the case where the decision tree is used as the model. Details of the random forest will be described later.

The neural network, the support vector machine, the decision tree, and the random forest, which will be described next, are used as preferable models or algorithms of supervised learning used by the learning units 103 and 203.

(4-1) Neural Network

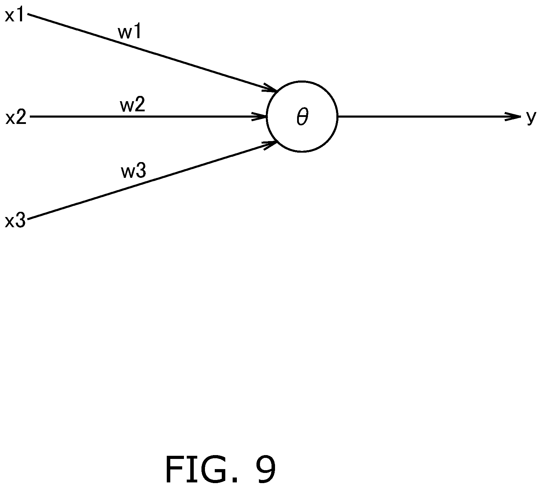

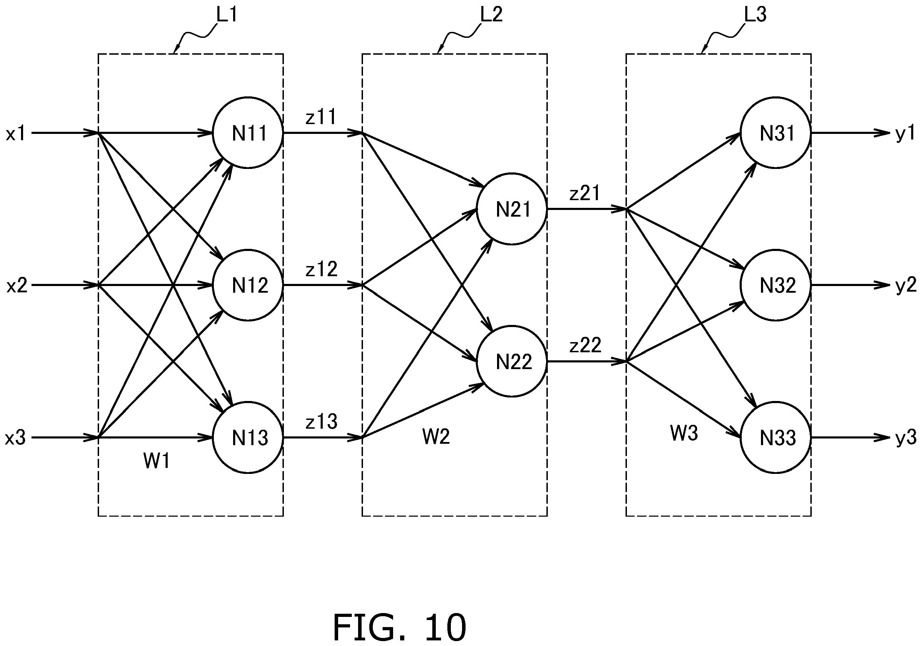

Fig. 9 is a schematic diagram of a model of a neuron in a neural network. Fig. 10 is a schematic diagram of a three-layer neural network constituted by a combination of the neurons illustrated in Fig. 9. As illustrated in Fig. 9, a neuron outputs an output y for a plurality of inputs x (inputs x1, x2, and x3 in Fig. 9). The inputs x (inputs x1, x2, and x3 in Fig. 9) are multiplied by corresponding weights w (weights w1, w2, and w3 in Fig. 9), respectively. The neuron outputs the output y by using Expression (1) below.

$$y = \varphi(\sum_{i=1}^{n} x_i w_i - \theta) \qquad (1)$$

In Expression (1), all of the inputs x, the output y, and the weights w are vectors, $\theta$ denotes a bias, and $\varphi$ denotes an activation function. The activation function is a non-linear function and is, for example, a step function (formal neuron), a simple perceptron, a sigmoid function, or a ReLU (ramp function).

In the three-layer neural network illustrated in Fig. 10, a plurality of input vectors x (input vectors x1, x2, and x3 in Fig. 10) are input from an input side (left side in Fig. 10), and a plurality of output vectors y (output vectors y1, y2, and y3 in Fig. 10) are output from an output side (right side in Fig. 10). This neural network is constituted by three layers L1, L2, and L3.

In the first layer L1, the input vectors x1, x2, and x3 are multiplied by corresponding weights and are input to each of three neurons N11, N12, and N13. In Fig. 10, these weights are collectively denoted by W1. The neurons N11, N12, and N13 output feature vectors z11, z12, and z13, respectively.

In the second layer L2, the feature vectors z11, z12, and z13 are multiplied by corresponding weights and are input to each of two neurons N21 and N22. In Fig. 10, these weights are collectively denoted by W2. The neurons N21 and N22 output feature vectors z21 and z22, respectively.

In the third layer L3, the feature vectors z21 and z22 are multiplied by corresponding weights and are input to each of three neurons N31, N32, and N33. In Fig. 10, these weights are collectively denoted by W3. The neurons N31, N32, and N33 output the output vectors y1, y2, and y3, respectively.

There are a learning mode and a prediction mode in terms of operation of the neural network. In the learning mode, the neural network learns the weights W1, W2, and W3 by using a learning dataset. In the prediction mode, the neural network performs prediction such as discrimination by using the parameters of the learned weights W1, W2, and W3.

The weights W1, W2, and W3 can be learned through error backpropagation (backpropagation), for example. In this case, information regarding the error is transferred from the output side toward the input side, that is, from the right side toward the left side in Fig. 10. The error backpropagation is a technique for performing learning by adjusting the weights W1, W2, and W3 such that a difference between the output y obtained when the input x is input to each neuron and the true output y (training data) decreases.

The neural network can be configured to have more than three layers. A machine learning technique using a neural network having four or more layers is known as deep learning.

(4-2) Support Vector Machine

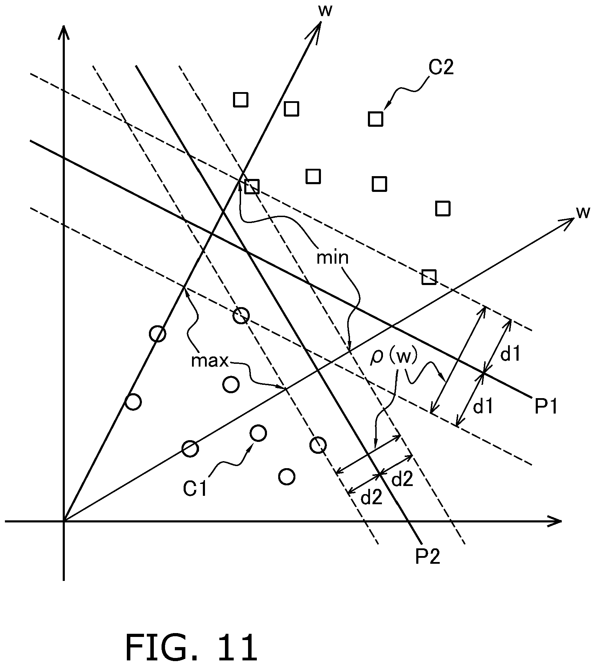

The support vector machine (SVM) is an algorithm that determines a two-class linear discriminant function that implements the maximum margin. Fig. 11 is a diagram for describing the SVM. The two-class linear discriminant function represents discrimination hyperplanes P1 and P2 which are hyperplanes for linearly separating pieces of learning data of two classes C1 and C2 from each other in a feature space illustrated in Fig. 11. In Fig. 11, pieces of learning data of the class C1 are represented by circles, and pieces of learning data of the class C2 are represented by squares. A margin of a discrimination hyperplane refers to a distance between learning data closest to the discrimination hyperplane and the discrimination hyperplane. Fig. 11 illustrates a margin d1 for the discrimination hyperplane P1 and a margin d2 for the discrimination hyperplane P2. In the SVM, the optimum discrimination hyperplane P1 which is a discrimination hyperplane with the maximum margin is determined. A minimum value d1 of the distance between the learning data of one class C1 and the optimum discrimination hyperplane P1 is equal to a minimum value d1 of the distance between the learning data of the other class C2 and the optimum discrimination hyperplane P1.

In Fig. 11, a learning dataset $D_L$ used in supervised learning of a two-class problem is represented by Expression (2) below.

$$D_L = \{(t_i, \boldsymbol{x}_i)\} \ (i = 1, \dots, N) \qquad (2)$$

The learning dataset $D_L$ is a set of pairs of learning data (feature vector) $x_i$ and training data ti = {-1, +1}. The number of elements of the learning dataset $D_L$ is N. The training data ti indicates which of the classes C1 and C2 the learning data $x_i$ belongs to. The class C1 is a class denoted by ti = -1, and the class C2 is a class denoted by ti = +1.

A normalized linear discriminant function that holds for all the pieces of learning data $x_i$ in Fig. 11 is represented by two Expressions (3-1) and (3-2) below. w denotes a coefficient vector and b denotes a bias.

If $t_i$ = +1 ,

$$\boldsymbol{w}^T \boldsymbol{x}_i + b \geq +1 \qquad (3-1)$$

If $t_i$ = -1,

$$\boldsymbol{w}^T \boldsymbol{x}_i + b \leq -1 \qquad (3-2)$$

These two Expressions are represented by one Expression (4) below.

$$t_i(\boldsymbol{w}^T \boldsymbol{x}_i + b) \geq 1 \qquad (4)$$

In the case where each of the discrimination hyperplanes P1 and P2 is represented by Expression (5) below, the margin d thereof is represented by Expression (6).

$$\boldsymbol{w}^T \boldsymbol{x} + b = 0 \qquad (5)$$

$$\mathrm{d} = \frac{1}{2}\rho(w) = \frac{1}{2}\left(\min_{x_i \in C_2} \frac{\boldsymbol{w}^T \boldsymbol{x}_i}{\|\boldsymbol{w}\|} - \max_{x_i \in C_1} \frac{\boldsymbol{w}^T \boldsymbol{x}_i}{\|\boldsymbol{w}\|}\right) \qquad (6)$$

In Expression (6), p(w) denotes the minimum value of a difference between lengths obtained by projecting the learning data $x_i$ of the class C1 and the learning data $x_i$ of the class C2 onto a normal vector w of the discrimination hyperplanes P1 and P2. The terms "min" and "max" in Expression (6) indicate points denoted by reference signs "min" and "max" in Fig. 11, respectively. In Fig. 11, the optimum discrimination hyperplane is the discrimination hyperplane P1 having the maximum margin d.

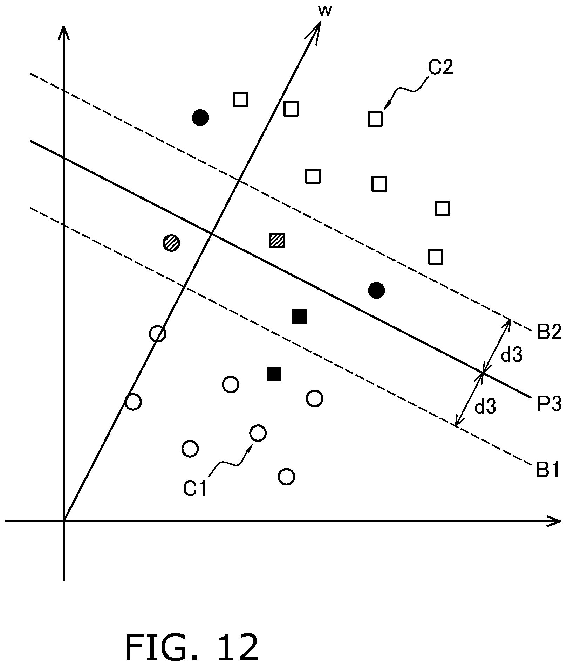

Fig. 11 illustrates the feature space in which the pieces of learning data of two classes are linearly separable. Fig. 12 illustrates a feature space which is similar to that of Fig. 11 and in which pieces of learning data of two classes are linearly inseparable. In the case where pieces of learning data of two classes are linearly inseparable, Expression (7) below, which is expanded by introducing a slack variable $\xi_i$ to Expression (4), can be used.

$$t_i(\boldsymbol{w}^T \boldsymbol{x}_i + b) - 1 + \xi_i \geq 0 \qquad (7)$$

The slack variable $\xi_i$ is used only at the time of learning and takes a value of 0 or greater. Fig. 12 illustrates a discrimination hyperplane P3, margin boundaries B1 and B2, and a margin d3. Expression for the discrimination hyperplane P3 is the same as Expression (5). The margin boundaries B1 and B2 are hyperplanes whose distance from the discrimination hyperplane P3 is the margin d3.

In the case where the slack variable $\xi_i$ is equal to 0, Expression (7) is equivalent to Expression (4). At this time, as indicated by blank circles or squares in Fig. 12, the learning data $x_i$ that satisfies Expression (7) is correctly discriminated within the margin d3. At this time, the distance between the learning data $x_i$ and the discrimination hyperplane P3 is greater than or equal to the margin d3.

In the case where the slack variable $\xi_i$ is greater than 0 and less than or equal to 1, as indicated by a hatched circle or square in Fig. 12, the learning data $x_i$ that satisfies Expression (7) is beyond the margin boundaries B1 and B2 but is not beyond the discrimination hyperplane P3 and thus is correctly discriminated. At this time, the distance between the learning data $x_i$ and the discrimination hyperplane P3 is less than the margin d3.

In the case where the slack variable $\xi_i$ is greater than 1, as indicated by black circles or squares in Fig. 12, the learning data $x_i$ that satisfies Expression (7) is beyond the discrimination hyperplane P3 and thus is incorrectly recognized.

The use of Expression (7) in which the slack variable $\xi_i$ is introduced enables the learning data $x_i$ to be discriminated in this manner also in the case where pieces of learning data of two classes are linearly inseparable.

From the description above, the sum of the slack variable $\xi_i$ for all the pieces of learning data $x_i$ indicates the upper limit of the number of pieces of learning data $x_i$ incorrectly recognized. Here, an evaluation function $L_p$ is defined by Expression (8) below.

$$L_p(w, \xi) = \frac{1}{2} w^T w + C \sum_{i=1}^{N} \xi_i \qquad (8)$$

The learning units 103 and 203 find a solution (w, $\xi$) that minimizes an output value of the evaluation function $L_p$. In Expression (8), a parameter C of the second term denotes a strength of a penalty for incorrect recognition. As the parameter C increases, a solution for prioritizing a reduction in the number of incorrect recognition (second term) over a norm (first term) of w is determined.

(4-3) Decision Tree

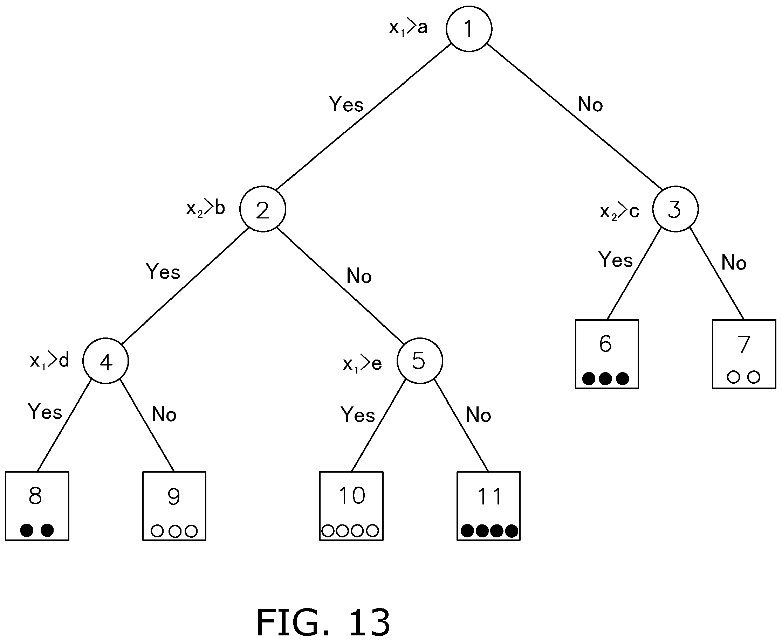

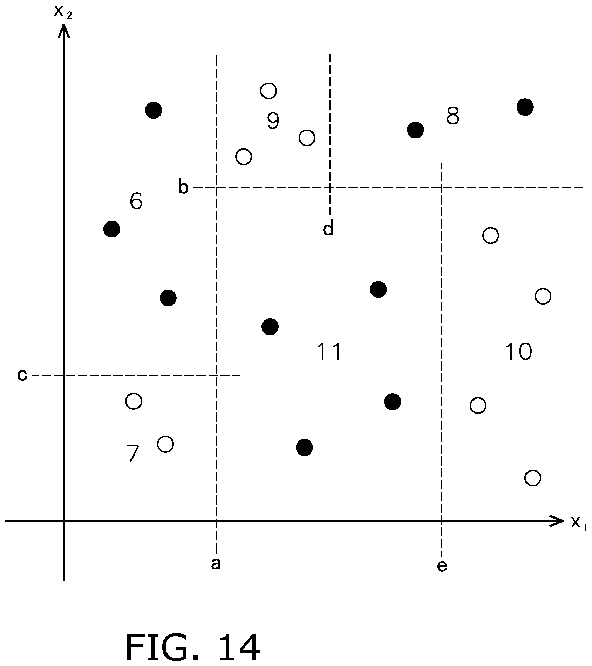

The decision tree is a model for obtaining a complex discrimination boundary (such as a non-linear discriminant function) by combining a plurality of discriminators. A discriminator is, for example, a rule regarding a magnitude relationship between a value on a certain feature axis and a threshold. Examples of a method for creating a decision tree from learning data include a divide and conquer algorithm for repeatedly finding a rule (discriminator) for dividing a feature space into two. Fig. 13 is an example of a decision tree created in accordance with the divide and conquer algorithm. Fig. 14 illustrates a feature space divided in accordance with the decision tree of Fig. 13. In Fig. 14, each piece of learning data is denoted by a white or black dot. Each piece of learning data is classified into a white dot class or a black dot class in accordance with the decision tree illustrated in Fig. 13. Fig. 13 illustrates nodes numbered from 1 to 11 and links, labeled Yes or No, linking the nodes to each other. In Fig. 13, a quadrangle denotes a terminal node (leaf node) and a circle denotes a non-terminal node (root node or internal node). The terminal nodes are nodes numbered from 6 to 11, and the non-terminal nodes are nodes numbered from 1 to 5. In each terminal node, white dots or black dots representing pieces of learning data are illustrated. Non-terminal nodes are equipped with respective discriminators. The discriminators are rules for determining a magnitude relationships between values on feature axes $x_1$ and $x_2$ and thresholds a to e. The labels assigned to the respective links indicate the determination results of the corresponding discriminators. In Fig. 14, the discriminators are represented by dotted lines, and a region divided by each of the discriminators is denoted by the numeral of the corresponding node.

In the process of creating an appropriate decision tree by using the divide and conquer algorithm, it is necessary to consider three points (a) to (c) below.

(a) Selection of a feature axis and a threshold for configuring a discriminator.
(b) Determination of a terminal node. For example, the number of classes to which the learning data included in one terminal node belongs. Alternatively, selection of how far decision tree pruning (obtaining subtrees having the same root node) is to be performed.
(c) Assignment of a class to a terminal node by a majority vote.

In a decision-tree-based learning method, for example, CART, ID3, and C4.5 are used. CART is a technique for generating a binary tree as a decision tree by dividing, for each feature axis, a feature space into two at each of nodes other than terminal nodes as illustrated in Figs. 13 and 14.

In learning using a decision tree, to improve the learning data discrimination performance, it is important to divide the feature space at an appropriate division candidate point at a non-terminal node. An evaluation function called a diversity index may be used as a parameter for evaluating the division candidate point of the feature space. As a function I(t) representing the diversity index of a node t, for example, parameters represented by Expressions (9-1) to (9-3) below are used. K denotes the number of classes.

(a) Error rate at node t

$$I(t) = 1 - \max_i P(C_i|t) \qquad (9-1)$$

(b) Cross-entropy (deviance)

$$I(t) = -\sum_{i=1}^{K} P(C_i|t) ln P(C_i|t) \qquad (9-2)$$

(c) Gini coefficient

$$I(t) = \sum_{i=1}^{K} \sum_{j \neq i} P(C_i|t) P(C_j|t) = \sum_{i=1}^{K} P(C_i|t)(1 - P(C_i|t)) \qquad (9-3)$$

In Expressions above, a probability $P(C_i|t)$ is a posterior probability of a class $C_i$ at the node t, that is, a probability of data of the class $C_i$ being selected at the node t. In the second part of Expression (9-3), a probability $P(C_j|t)$ is a probability of data of the class $C_i$ being incorrectly discriminated to be in a j-th ($\neq$ i-th) class. Thus, the second part represents an error rate at the node t. The third part of Expression (9-3) represents a sum of variances of the probability $P(C_i|t)$ for all the classes.

In the case of dividing a node by using the diversity index as the evaluation function, for example, a technique of pruning the decision tree up to an allowable range that is determined by an error rate at the node and by the complexity of the decision tree is used.

(4-4) Random Forest

The random forest is a type of ensemble learning and is a technique for enhancing the discrimination performance by combining a plurality of decision trees. In learning using the random forest, a group (random forest) of a plurality of decision trees having a low correlation is generated. The following algorithm is used in generation of the random forest and discrimination using the random forest.

(A) The following is repeated while m = 1 to M.

(a) From N pieces of d-dimensional learning data, m bootstrap samples $Z_m$ are generated.
(b) By using $Z_m$ as learning data, each node t is divided in the following procedure to generate m decision trees.

(i) From d features, d' features are randomly selected. (d' < d)
(ii) From among the d' selected features, a feature that implements optimum division of the learning data and a division point (threshold) are determined.
(iii) The node t is divided into two at the determined division point.

(B) A random forest constituted by the m decision trees is output.
(C) A discrimination result of each decision tree of the random forest for input data is obtained. A discrimination result of the random forest is determined by a majority vote of the discrimination results of the respective decision trees.

In learning using the random forest, a correlation between decision trees can be made low by randomly selecting a predetermined number of features for use in discrimination at individual non-terminal nodes of the decision trees.

(5) Modification E

Reinforcement learning that is a machine learning technique used by the learning units 103 and 203 in the first to third embodiments will be described. Reinforcement learning is a technique for learning a policy that maximizes a reward which is a result of a series of actions. Models or algorithms used in reinforcement learning include Q-learning or the like. Q-learning is a technique for learning a Q-value that represents a value of selecting an action a in a state s. In Q-learning, an action a with the highest Q-value is selected as an optimum action. To determine a high Q-value, an entity (agent) of the action a is rewarded for the action a selected in the state s. In Q-learning, the Q-value is updated by using Expression (10) below every time the agent takes an action.

$$Q(s_t, a_t) \leftarrow Q(s_t, a_t) + \alpha \left( r_{t+1} + \gamma \max_\alpha Q(s_{t+1}, a_t) - Q(s_t, a_t) \right) \qquad (1\ 0)$$

In Expression (10), Q(st, at) is the Q-value that represents a value of the agent in a state $s_t$ selecting an action at. Q(st, at) is a function (action-value function) having a state s and an action a as parameters. $s_t$ denotes a state of the agent at a time t. at denotes an action of the agent at the time t. $\alpha$ denotes a learning coefficient. $\alpha$ is set such that the Q-value converges to an optimum value in accordance with Expression (10). $r_{t+1}$ denotes a reward obtained when the agent transitions to a state $s_{t+1}$. $\gamma$ denotes a discount factor. $\gamma$ is a constant that is greater than or equal to 0 and less than or equal to 1. The term including max is a product obtained by multiplying by y the Q-value in the case of selecting the action a with the highest Q-value in the state $s_{t+1}$. The Q-value determined by using the action-value function is an expected value of the reward to be obtained by the agent.

(6) Modification F

In the third embodiment, the machine learning device 200 includes the control amount acquisition unit 202. However, the machine learning device 200 need not include the control amount acquisition unit 202. In this case, the learning unit 203 of the machine learning device 200 may use, as the learning data, the control parameter determined by the control amount determining unit 206.

(7) Modification G

In the embodiments and modifications described above, the machine learning devices 100 and 200 use supervised learning or reinforcement learning. However, the machine learning devices 100 and 200 may use a combination technique of supervised learning and reinforcement learning.

(8) Modification H

In the embodiments and modifications described above, the learning units 103 and 203 may use various machine learning techniques. Machine learning techniques that may be used by the learning units 103 and 203 include unsupervised learning, semi-supervised learning, transductive learning, multi-task learning, transfer learning, etc. in addition to supervised learning and reinforcement learning already described. The learning units 103 and 203 may use these techniques in combination.

Unsupervised learning is a technique of grouping (clustering) input data on the basis of a predetermined statistical property without using training data. Models or algorithms used in unsupervised learning include k-means clustering, the Ward's method, the principal component analysis, etc. The k-means clustering is a technique in which a process of randomly assigning a cluster to each piece of input data, calculating the center of each cluster, and re-assigning each piece of input data to a cluster having the nearest center is repeated. The Ward's method is a technique in which a process of assigning each piece of input data to a cluster is repeated to minimize a distance from each piece of input data of a cluster to the mass center of the cluster. The principal component analysis is a technique of a multivariate analysis that generates variables called principal components having the lowest correlation from among a plurality of correlated variables.

The semi-supervised learning is a technique of performing learning by using both input data (unlabeled data) not assigned corresponding training data and input data (labeled data) assigned corresponding training data.

The transductive learning is a technique of generating an output corresponding to unlabeled data for use in learning and not generating an output corresponding to unseen input data in semi-supervised learning.

The multi-task learning is a technique of sharing information among a plurality of related tasks and causing these tasks to simultaneously perform learning to obtain a factor that is common to the tasks and increase the prediction accuracy.

The transfer learning is a technique of applying a model trained in advance in a certain domain to another domain to increase the prediction accuracy.

< In Closing >

While the embodiments of the present disclosure have been described above, it should be understood that various modifications can be made on the configurations and details without departing from the gist and the scope of the present disclosure that are described in the claims.

Industrial Applicability

The machine learning device can acquire a predicted value of the thermal sensation of a subject with a high accuracy.

**REFERENCE SIGNS LIST**

| 10 | environment adjusting apparatus |
| 20 | subject |
| 100 | machine learning device |
| 101 | state variable acquisition unit (first acquisition unit) |
| 102 | control amount acquisition unit (second acquisition unit) |
| 103 | learning unit |
| 104 | function updating unit (updating unit) |
| 105 | inference unit |
| 106 | operation amount candidate output unit (output unit) |
| 107 | operation amount determining unit (determining unit) |
| 200 | machine learning device |
| 201 | state variable acquisition unit (first acquisition unit) |
| 202 | control amount acquisition unit (second acquisition unit) |
| 203 | learning unit |
| 204 | function updating unit (updating unit) |
| 204a | training data accumulation unit (accumulation unit) |
| 204b | assessment unit |
| 205 | evaluation data acquisition unit (third acquisition unit) |
| 207 | function altering unit (altering unit) |

**CITATION LIST**

**PATENT LITERATURE**

PTL 1: International Publication No. 2007/007632

**Claims**

1. A machine learning device (100) configured to learn a thermal sensation of a subject (20), comprising:

   a first acquisition unit (101) configured to acquire a first variable including a parameter related to biological information of the subject;
   a second acquisition unit (102) configured to acquire a second variable including a thermal sensation of the subject; and
   a learning unit (103) configured to learn the first variable and the second variable in association with each other.

2. The machine learning device according to Claim 1, wherein
   the first variable includes at least one of parameters correlated to a brain wave, a skin blood flow rate, a skin temperature, an amount of sweat, and a heartbeat of the subject.

3. The machine learning device according to Claim 1 or 2, wherein
   the learning unit performs learning by using, as training data, the first variable and the second variable.

4. The machine learning device according to any one of Claims 1 to 3, further comprising:
   an inference unit (105) configured to infer a predicted value of the thermal sensation of the subject from the first variable on the basis of a learning result of the learning unit.

5. The machine learning device according to Claim 4, further comprising:

an updating unit (104) configured to calculate a reward on the basis of the second variable and the predicted value, wherein
the learning unit performs learning by using the reward.

6. The machine learning device according to Claim 5, wherein
the updating unit calculates a higher reward as a difference between the thermal sensation of the subject included in the second variable and the predicted value becomes smaller.

7. An environment adjusting apparatus configured to adjust an environment in a target space, comprising: the machine learning device according to any one of Claims 1 to 6.

8. The environment adjusting apparatus according to Claim 7, wherein
the second acquisition unit acquires the second variable on the basis of at least one of a value related to the thermal sensation input by the subject and an operation situation of the environment adjusting apparatus.

9. The environment adjusting apparatus according to Claim 7 or 8, comprising:

an output unit (106) configured to output candidates for a third variable for use in adjusting the environment in the target space; and
a determining unit (107) configured to determine the third variable, wherein
the machine learning device is the machine learning device according to any one of Claims 4 to 6;
the inference unit of the machine learning device infers the predicted value on the basis of the candidates output by the output unit, and
the determining unit determines the third variable such that the predicted value satisfies a predetermined condition.

10. The environment adjusting apparatus according to Claim 9, wherein

the determining unit determines the third variable such that a difference between a target value of the thermal sensation of the subject and the predicted value inferred by the inference unit decreases, and
the learning unit performs learning by using the third variable determined by the determining unit.

11. The environment adjusting apparatus according to Claim 9 or 10, wherein
the third variable includes a temperature in the target space.

12. A machine learning device configured to learn a control parameter of an environment adjusting apparatus (10) configured to adjust an environment in a target space, the machine learning device comprising:

a first acquisition unit (201) configured to acquire a first variable including a parameter related to biological information of a subject in the target space;
a second acquisition unit (202) configured to acquire the control parameter; and
a learning unit (203) configured to learn the first variable and the control parameter in association with each other.

13. The machine learning device according to Claim 12, further comprising:

a third acquisition unit (205) configured to acquire evaluation data for evaluating a control result of the environment adjusting apparatus; and
an updating unit (204) configured to update, by using the evaluation data, a learning state of the learning unit, wherein
the learning unit performs learning in accordance with an output of the updating unit, and
the evaluation data includes a thermal sensation of the subject.

14. The machine learning device according to Claim 13, wherein

the updating unit calculates a reward on the basis of the evaluation data, and
the learning unit performs learning by using the reward.

15. The machine learning device according to Claim 14, wherein

the evaluation data is a difference between a predicted value of the thermal sensation of the subject and a neutral value of a thermal sensation, and

the updating unit calculates a higher reward as the difference becomes smaller.

16. The machine learning device according to Claim 13, further comprising:

an altering unit (207) configured to output a parameter of a discriminant function whose input variable is the first variable and whose output variable is the control parameter, wherein

the learning unit alters the parameter of the discriminant function in accordance with an output of the altering unit a plurality of times and outputs, for each discriminant function whose parameter has been altered, the control parameter from the first variable,

the updating unit comprises an accumulation unit (204a) and an assessment unit (204b),

the assessment unit outputs an assessment result by using the evaluation data,

the accumulation unit accumulates, in accordance with the assessment result, training data based on the first variable and the control parameter output by the learning unit from the first variable, and

the learning unit performs learning on the basis of the training data accumulated in the accumulation unit.

17. The machine learning device according to any one of Claims 13 to 16, wherein

the third acquisition unit acquires the evaluation data on the basis of at least one of a value related to the thermal sensation input by the subject and an operation situation of the environment adjusting apparatus.

18. The machine learning device according to any one of Claims 12 to 17, wherein

the first variable includes at least one of parameters correlated to a brain wave, a skin blood flow rate, a skin temperature, and an amount of sweat of the subject.

19. An environment adjusting apparatus comprising: the machine learning device according to any one of Claims 12 to 18.

**FIG. 1**

FIG. 2

FIG. 3

EP 4 067 769 A1

FIG. 4

FIG. 5

FIG. 6

FIG. 7

FIG. 8

EP 4 067 769 A1

FIG. 9

EP 4 067 769 A1

FIG. 10

FIG. 11

FIG. 12

FIG. 13

FIG. 14

EP 4 067 769 A1

| | INTERNATIONAL SEARCH REPORT | International application No. |
|---|---|---|
| | | PCT/JP2020/044112 |

A. CLASSIFICATION OF SUBJECT MATTER
Int.Cl. F24F11/62(2018.01)i, F24F11/64(2018.01)i, F24F11/72(2018.01)i,
F24F11/80(2018.01)i, F24F110/10(2018.01)n, F24F120/14(2018.01)n,
F24F120/20(2018.01)n, F24F140/00(2018.01)n, G05B13/02(2006.01)i,
A61B5/01(2006.01)i
FI: F24F11/62, G05B13/02L, G05B13/02B, A61B5/01100, F24F11/64, F24F11/72,
F24F11/80, F24F120:20, F24F110:10, F24F140:00, F24F120:14
According to International Patent Classification (IPC) or to both national classification and IPC

B. FIELDS SEARCHED

Minimum documentation searched (classification system followed by classification symbols)
Int.Cl. F24F11/62, F24F11/64, F24F11/72, F24F11/80, F24F110/10,
F24F120/14, F24F120/20, F24F140/00, G05B13/02, A61B5/01

Documentation searched other than minimum documentation to the extent that such documents are included in the fields searched
Published examined utility model applications of Japan 1922–1996
Published unexamined utility model applications of Japan 1971–2021
Registered utility model specifications of Japan 1996–2021
Published registered utility model applications of Japan 1994–2021

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2019-082312 A (DAIKIN INDUSTRIES, LTD.) 30 May 2019 (2019-05-30), paragraphs [0038]-[0134], fig. 1-9 | 1, 12-13 |
| Y | | 2-11, 14-19 |
| Y | JP 09-303842 A (TOSHIBA CORPORATION) 28 November 1997 (1997-11-28), paragraphs [0026]-[0031], fig. 1-3 | 2-11, 18-19 |
| Y | JP 2009-228918 A (HITACHI, LTD.) 08 October 2009 (2009-10-08), paragraph [0121] | 5-11, 14-19 |

☐ Further documents are listed in the continuation of Box C.  ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 21 January 2021 | 02 February 2021 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**
Information on patent family members

International application No.

PCT/JP2020/044112

| | | | |
|---|---|---|---|
| JP 2019-082312 A | 30 May 2019 | WO 2019/087537 A1<br>paragraphs [0038]-[0134],<br>fig. 1-9 | |
| JP 09-303842 A | 28 November 1997 | (Family: none) | |
| JP 2009-228918 A | 08 October 2009 | (Family: none) | |

Form PCT/ISA/210 (patent family annex) (January 2015)

**EP 4 067 769 A1**

**REFERENCES CITED IN THE DESCRIPTION**

**Patent documents cited in the description**

- WO 2007007632 A